(19) Europäisches Patentamt
European Patent Office
Office européen des brevets

(11) **EP 3 040 096 B1**

(12) **FASCICULE DE BREVET EUROPEEN**

(45) Date de publication et mention
de la délivrance du brevet:
**17.04.2019 Bulletin 2019/16**

(51) Int Cl.:
***A61M 16/00*** *(2006.01)*    ***A61M 16/12*** *(2006.01)*
***A61M 16/20*** *(2006.01)*

(21) Numéro de dépôt: **15197031.6**

(22) Date de dépôt: **30.11.2015**

(54) **APPAREIL D'ASSISTANCE RESPIRATOIRE AVEC DÉTECTION DE TOUT ARRÊT DE LA TURBINE**

GERÄT ZUR ATMUNGSUNTERSTÜTZUNG MIT STILLSTANDSERFASSUNG DER TURBINE

BREATHING ASSISTANCE APPARATUS WITH DETECTION OF ANY HALTING OF THE TURBINE

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **05.01.2015 FR 1550023**

(43) Date de publication de la demande:
**06.07.2016 Bulletin 2016/27**

(73) Titulaire: **Air Liquide Medical Systems
92160 Antony (FR)**

(72) Inventeur: **BOULANGER, Thierry
Swarthmore, PA 19081 (US)**

(74) Mandataire: **Pittis, Olivier
L'Air Liquide, S.A.
Direction de la Propriété Intellectuelle
75, Quai d'Orsay
75321 Paris Cedex 07 (FR)**

(56) Documents cités:
**WO-A1-00/21596          WO-A1-00/45883
WO-A1-2012/032434     FR-A1- 2 830 454
US-A- 5 694 926          US-A- 5 701 883
US-A1- 2010 078 017   US-A1- 2013 008 444**

**Description**

La présente invention concerne un ventilateur ou appareil médical d'assistance respiratoire à turbine motorisée et moyens de pilotage comprenant un organe d'obturation, agencé sur le conduit d'amenée d'air et piloté par les moyens de pilotage du ventilateur, permettant d'empêcher toute sortie d'oxygène par l'orifice d'entrée d'air, en cas d'arrêt du fonctionnement de la turbine, encore appelée « soufflante », « micro-soufflante » ou « compresseur » .

Certaines pathologies respiratoires nécessitent l'administration aux patients qui en sont atteints, d'air ou d'un mélange d'air enrichi en oxygène.

Une telle administration de gaz peut être réalisée au moyen d'un appareil d'assistance respiratoire, couramment appelé ventilateur médical, équipé d'une turbine à moteur électrique, encore appelée compresseur d'air ou soufflante.

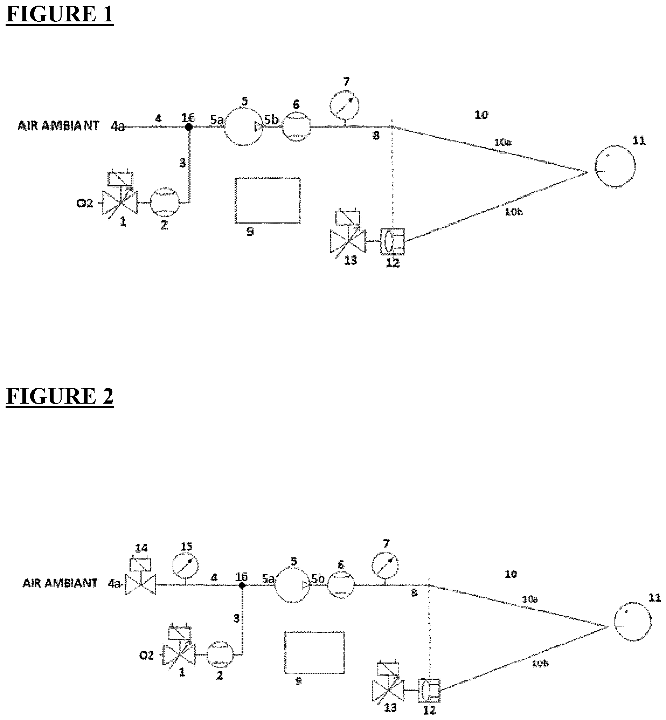

D'une façon générale, la turbine sert à générer un flux de gaz à pression donnée et contenant une proportion souhaitée d'oxygène, à savoir 21% ou plus d'oxygène (% en vol.). Le principe de fonctionnement d'un tel ventilateur de l'art antérieur est illustré sur la Figure 1 et détaillé ci-après.

Habituellement, la turbine est alimentée en air par un conduit d'amenée d'air relié, d'une part, à l'atmosphère ambiant et, d'autre part, à l'entrée d'aspiration de la turbine et, par ailleurs, en oxygène, par un conduit d'alimentation en oxygène alimenté par une source d'oxygène et venant se raccorder au conduit d'amenée d'air, en amont de la turbine. De manière classique, une électrovanne proportionnelle agencée sur le conduit d'alimentation en oxygène permet de contrôler la quantité d'oxygène délivré par la source d'oxygène.

Toutefois, lorsque l'on souhaite maintenir une ventilation du patient alors que la turbine est arrêtée, que ce soit volontairement pour des questions d'économie d'énergie dans le cadre d'un transport long par exemple, ou involontairement du fait d'une défaillance de la turbine, le débit d'oxygène délivré par l'électrovanne agencée sur le conduit d'alimentation en oxygène ne peut atteindre le patient car il s'échappe à l'atmosphère ambiant en remontant la canalisation d'amenée d'air, étant donné que la turbine qui est arrêtée, n'aspire plus d'air.

Le patient n'est alors plus alimenté en oxygène.

On connaît en outre le document US-A-2013/008444 qui enseigne un appareil d'assistance respiratoire à turbine alimentée par un conduit d'amenée d'air avec un orifice d'entrée d'air communiquant fluidiquement avec l'atmosphère ambiant. Dans l'un des modes de réalisation, un conduit d'alimentation en oxygène vient se raccorder au conduit d'amenée d'air en amont de l'entrée de gaz de la turbine. Des moyens de pilotage coopèrent avec la turbine et un organe d'obturation de conduit est agencé sur le conduit d'amenée d'air entre l'orifice d'entrée d'air et le raccordement du conduit d'alimentation en oxygène. Cette architecture vise à contrôler les flux de gaz de manière à éviter les flux inverses de gaz.

On connait en outre les documents FR-A-2830454, WO-A-00/45883, WO-A-2012/032434, US-A-2010/0787017 et US-A-5694926 décrivant d'autres appareils d'assistance respiratoire connus.

Le problème qui se pose est dès lors d'améliorer ce type de ventilateur à turbine pour permettre de maintenir une ventilation du patient même lorsque la turbine est arrêtée, c'est-à-dire de pouvoir acheminer l'oxygène issu de la source d'oxygène jusqu'au patient même lorsque la turbine ne fonctionne pas.

La solution de l'invention est alors un ventilateur médical à turbine, c'est-à-dire un appareil d'assistance respiratoire comprenant :

- une turbine à moteur électrique comprenant une entrée de gaz et une sortie de gaz, la sortie de gaz de la turbine étant en communication fluidique avec un circuit patient, la sortie de gaz de la turbine étant reliée fluidiquement au circuit patient par l'intermédiaire d'une ligne d'alimentation en gaz,
- un conduit d'amenée d'air comprenant un orifice d'entrée d'air communiquant fluidiquement avec l'atmosphère ambiant, ledit conduit d'amenée d'air par ailleurs relié fluidiquement à l'entrée de gaz de la turbine,
- un conduit d'alimentation en oxygène venant se raccorder au conduit d'amenée d'air, en un site de raccordement situé en amont de l'entrée de gaz de la turbine, le conduit d'alimentation en oxygène étant en communication fluidique avec le conduit d'amenée d'air, et
- des moyens de pilotage coopérant avec la turbine de manière à pouvoir détecter tout arrêt de fonctionnement de ladite turbine, et
- un organe d'obturation de conduit agencé sur le conduit d'amenée d'air entre l'orifice d'entrée d'air et le site de raccordement du conduit d'alimentation en oxygène au conduit d'amenée d'air,

caractérisé en ce que :

- les moyens de pilotage sont configurés pour détecter un arrêt du fonctionnement de la turbine et pour piloter l'organe d'obturation de conduit de manière à obturer le conduit d'amenée d'air et à empêcher toute circulation de gaz vers l'orifice d'entrée d'air lorsque lesdits moyens de pilotage détectent un arrêt du fonctionnement de la turbine,
- une première électrovanne est agencée sur le conduit d'alimentation en oxygène,

- la ligne d'alimentation en gaz comprend un capteur de débit et un capteur de pression,
- et les moyens de pilotage sont en outre configurés pour commander la première électrovanne, en cas d'arrêt de la turbine, en réponse à des signaux provenant du capteur de débit ou du capteur de pression de manière à ajuster le débit d'oxygène circulant dans ledit conduit d'alimentation en oxygène en alternant :

  - des phases de délivrance de gaz durant lesquelles de l'oxygène peut traverser la première électrovanne à un débit de délivrance (D) donné, et
  - des phases de non-délivrance de gaz durant lesquelles de l'oxygène ne traverse pas la première électrovanne ou la traverse à un débit limité (d).

Selon le cas, le ventilateur médical ou appareil d'assistance respiratoire de l'invention peut comprendre l'une ou plusieurs des caractéristiques techniques suivantes :

- typiquement, on a d < D.
- l'organe d'obturation de conduit comprend un actionneur, de préférence un actionneur à deux positions de fonctionnement, à savoir une position ouverte et une position fermée. Tout autre dispositif fonctionnant sur le principe ouvert/fermé peut être utilisé.
- l'organe d'obturation de conduit comprend un actionneur à deux positions de fonctionnement comprenant une position ouverte dans laquelle le gaz peut circuler dans le conduit d'amenée d'air, et une position fermée dans lequel le gaz ne peut pas circuler dans le conduit d'amenée d'air et sortir dudit conduit d'amenée d'air par l'orifice d'entrée d'air.
- les moyens de pilotage pilotent la première électrovanne de manière à alterner des phases de délivrance de gaz durant lesquelles de l'oxygène peut traverser la première électrovanne à un débit de délivrance donné D, de préférence entre environ 0 et 200 L/min, et des phases de non-délivrance de gaz durant lesquelles de l'oxygène ne traverse pas la première électrovanne (i.e. débit 0 L/mn) ou la traverse à débit limité d pouvant atteindre environ 10 L/min, i.e. un débit limité d qui est inférieur ou égal à 10 L/min.
- lors des phases de non-délivrance de gaz, les moyens de pilotage pilotent la première électrovanne de manière à ce que de l'oxygène traverse la première électrovanne à un débit limité d compris entre environ 1 et 10 L/min.
- lors des phases de délivrance de gaz, les moyens de pilotage pilotent la première électrovanne de manière à ce que de l'oxygène traverse la première électrovanne à un débit de délivrance D sélectionné en fonction des réglages du ventilateur ; il peut atteindre 200 l/min.
- le circuit patient alimente une interface respiratoire, tel qu'un masque, une sonde trachéale ou des lunettes respiratoires, qui est conçue pour fournir du gaz respiratoire au patient.
- le conduit d'alimentation en oxygène comprend un capteur de débit additionnel.
- le conduit d'amenée d'air comprend un capteur de pression additionnel.
- les moyens de pilotage commandent la turbine.
- les moyens de pilotage comprennent au moins une carte électronique de commande.
- les moyens de pilotage comprennent au moins une carte électronique de commande comprenant au moins un microprocesseur, typiquement de type microcontrôleur, mettant en oeuvre au moins algorithme.
- les moyens de pilotage comprennent au moins une carte mémoire permettant de stocker/mémoriser des données, c'est-à-dire tout type d'information.
- la carte électronique de commande est configurée pour traiter des signaux provenant des capteurs de débit et de pression.
- la première électrovanne est agencée sur le conduit d'alimentation en oxygène, entre l'entrée d'oxygène du conduit d'alimentation en oxygène à laquelle vient se raccorder fluidiquement une source d'oxygène, et le capteur de débit du conduit d'alimentation en oxygène.
- des moyens d'alimentation électrique, tel que câbles, transformateur, prise électrique..., permettant d'alimenter l'appareil, en particulier la carte électronique des moyens de pilotage, en courant électrique.

L'invention concerne également une installation de fourniture de gaz à un patient comprenant un appareil d'assistance respiratoire à turbine électrique selon l'invention, caractérisée en ce que le circuit patient est relié fluidiquement à une interface respiratoire, en particulier un masque, une sonde trachéale ou des lunettes respiratoires.

Selon le cas, l'installation de fourniture de gaz de l'invention peut comprendre l'une ou plusieurs des caractéristiques techniques suivantes :

- le circuit patient comprend une branche inspiratoire et une branche expiratoire comprenant une vanne expiratoire à membrane expiratoire commandée par une seconde électrovanne, ladite seconde électrovanne étant pilotée par les moyens de pilotage.

- la seconde électrovanne est de type proportionnel.
- le conduit d'alimentation en oxygène comprend une entrée d'oxygène raccordée fluidiquement à une source d'oxygène, de préférence une bouteille de gaz, une canalisation ou un réseau de canalisations véhiculant de l'oxygène, ou autre.

La présente description concerne aussi une utilisation de l'appareil de ventilation de l'invention pour traiter une pathologie respiratoire affectant un patient humain, laquelle pathologie nécessite ou requiert une administration d'air ou d'un mélange d'air enrichi en oxygène audit patient atteint de ladite pathologie respiratoire.

L'invention va maintenant être mieux comprise grâce à la description détaillée suivante, faite à titre illustratif mais non limitatif, en référence aux figures annexées parmi lesquelles :

- la Figure 1 illustre l'architecture et le principe de fonctionnement d'un ventilateur médical à turbine selon l'art antérieur,
- la Figure 2 illustre l'architecture et le principe de fonctionnement d'un ventilateur médical à turbine selon la présente invention, et
- la Figure 3 représente le ventilateur de la Figure 2 en phase d'autodiagnostic.

La Figure 1 est un schéma de l'architecture et du principe de fonctionnement d'un appareil d'assistance respiratoire ou ventilateur médical à turbine 5 à moteur électrique selon l'art antérieur.

La turbine 5 comprend une entrée de gaz 5a par laquelle le gaz est aspiré par la turbine 5 lors de son fonctionnement et une sortie de gaz 5b, la sortie de gaz 5b étant en communication fluidique avec le circuit patient 10 du ventilateur de manière à y délivrer le gaz respiratoire destiné au patient 11.

De façon classique, la turbine 5 aspire l'air ambiant présent dans l'atmosphère via un conduit d'amenée d'air 4 relié à l'entrée de la turbine 5. L'air ambiant pénètre dans le conduit d'amenée d'air 4 par un orifice d'entrée d'air 4a qui communique fluidiquement avec l'atmosphère ambiant.

De manière connue en soi, l'aspiration du gaz par la turbine 5 se fait au moyen d'une roue à ailettes mise en rotation par le moteur électrique de la turbine, lors de son fonctionnement.

L'air peut être additionné d'oxygène provenant d'une source d'oxygène ($O_2$), via un conduit d'alimentation 3 en oxygène venant se raccorder au conduit d'air 4, en un site de raccordement situé en amont de la turbine 5, c'est-à-dire entre l'entrée de gaz 5a de la turbine et l'orifice d'entrée d'air 4a du conduit d'air 4.

Le conduit d'alimentation 3 en oxygène comprend classiquement un premier capteur de débit 2 et une première électrovanne proportionnelle 1 située entre la source d'oxygène et le premier capteur 2, alors que la ligne d'alimentation en gaz 8 située en sortie de la turbine 5 comprend classiquement un second capteur de débit 6 et un capteur de pression 7.

La source d'oxygène peut être une bouteille d'oxygène, une canalisation ou un réseau véhiculant de l'oxygène ou autre. La source d'oxygène vient se raccorder à l'entrée du conduit d'alimentation 3 en oxygène.

Les capteurs de débit 2, 6 permettent de mesurer les débits de gaz circulant dans le conduit d'alimentation 3 en oxygène et dans la ligne d'alimentation en gaz 8, alors que le capteur de pression 7 permet de mesurer la pression gazeuse dans la ligne 8.

Les mesures de débit opérées par ces capteurs 2, 6 sont transmises aux moyens de pilotage 9 du ventilateur, telle une carte électronique de commande, qui les utilise comme expliqué ci-après.

La carte électronique de commande des moyens de pilotage 9 comprend classiquement un ou plusieurs microprocesseurs qui traitent notamment les informations issues des différents capteurs et permettent en conséquence le pilotage des actionneurs.

La turbine 5 sert à augmenter la pression de l'air ou du mélange air/$O_2$ à une valeur de pression supérieure à la pression atmosphérique (i.e. > 1 bar abs) et le flux gazeux sous pression ainsi obtenu est envoyé ensuite vers les voies respiratoires d'un patient 11 souffrant d'une maladie respiratoire de manière à l'aider à respirer. La turbine 5 délivre donc soit de l'air, soit un mélange gazeux air/$O_2$.

Lorsqu'il s'agit d'un mélange air/$O_2$, le mélange est réalisé en considérant :

- d'une part, la concentration souhaitée en oxygène, encore appelée fraction inspirée d'oxygène ou $FiO_2$, c'est-à-dire la teneur en oxygène du mélange final administré au patient 11, et
- d'autre part, la nature des gaz servant à réaliser le mélange gazeux, sachant que l'air ambiant contient 21% en volume d'$O_2$ et que la source d'$O_2$ pressurisé contient en général de l'oxygène pur, c'est-à-dire à une teneur de 100% en volume.

Comme mentionné ci-avant, la carte de commande 9 du ventilateur traite les signaux issus des capteurs de débit 2 et 6, pour obtenir un mélange air/$O_2$ correspondant à la $FiO_2$ souhaitée, en appliquant la Formule de calcul (1) suivante :

$$QO2 = Qtot*(fiO2 - 0.21)/0.79 \qquad (1)$$

où :

- QO2 est le débit d'oxygène (l/min) mesuré par le premier capteur de débit 2 et transformé par la carte de commande 9,
- Qtot est le débit total (l/min) délivré par la turbine mesuré par le deuxième capteur de débit 6 et transformé) par la carte de commande 9,
- FiO2 est la concentration souhaitée en oxygène (% en vol.).

Pour ce faire, la carte de commande 9 pilote la première électrovanne proportionnelle 1 pour que celle-ci laisse passer plus ou moins d'oxygène dans le conduit d'alimentation 3 en oxygène de sorte que l'équation de la Formule (1) soit en permanence vérifiée.

Le mélange air/$O_2$ ainsi réalisé et délivré pendant les phases inspiratoires du patient 11.

Lors de chaque phase inspiratoire du patient 11, la turbine 5 délivre au patient 11 soit un volume de gaz, dans le cadre d'une ventilation volumétrique, soit une pression gazeuse dans le cadre d'une ventilation barométrique.

Dans le cadre d'une ventilation volumétrique, le capteur de débit 6 renvoie à la carte de commande 9 une information en débit et la carte de commande 9 pilote alors la turbine 5 afin que celle-ci ajuste son débit de sortie en conséquence. Le gaz délivré emprunte la canalisation 8 puis la branche inspiratoire 10a du circuit patient 10, avant d'être administré au patient 11, via une interface respiratoire adaptée, tel un masque ou analogue.

Le circuit patient 10 comprend en outre une branche expiratoire 10b comprenant, de manière classique, une vanne expiratoire à membrane expiratoire 12 commandée par une seconde électrovanne 13 de type proportionnel.

Lors des phases inspiratoires, afin que le mélange gazeux air/$O_2$ ne puisse s'échapper à l'atmosphère par la vanne expiratoire et puisse être inhalé par le patient 11, la carte de commande 9 actionne par ailleurs la seconde électrovanne 13 afin que la membrane expiratoire 12 de la vanne expiratoire soit maintenue en position fermée. La branche expiratoire 10b du circuit patient 10 est alors obstruée.

Dans le cadre d'une ventilation barométrique, le capteur de pression 7 se substitue au second capteur de débit 6 et la carte de commande 9 traite alors les signaux de mesure de pression émanant dudit capteur de pression 7 pour piloter en conséquence la turbine 5.

A l'inverse, lors de chaque phase expiratoire, la carte de commande 9 commande la seconde électrovanne 13 qui agit alors sur la membrane 12 pour la faire passer en position ouverte et permettre ainsi une circulation du gaz dans la branche expiratoire 10b du circuit patient 10 et ensuite son évacuation à l'atmosphère, à savoir du gaz expiré par le patient 11 qui est chargé en $CO_2$.

Il est à noter qu'à des fins thérapeutiques, la carte de commande 9 peut aussi exploiter le signal de pression renvoyé par le capteur 7 afin de piloter la seconde électrovanne 13 de façon proportionnelle, et par conséquent, la membrane 12 et garantir que l'expiration se fasse à une pression supérieure à la pression ambiante ; on parle alors de pression expiratoire positive ou PEP.

Dans le même temps, la carte de commande 9 pilote la turbine 5 de telle manière à délivrer un débit de gaz constant, appelé débit de biais, en exploitant le signal du deuxième capteur de débit 6. Le débit de biais est par exemple compris entre 1 et 10 l/min ; il permet notamment d'éviter l'accumulation de $CO_2$ dans le circuit patient 10.

Par ailleurs, des moyens d'alimentation électrique classiques, pouvant comprendre un ou des câbles, transformateur de courant, prise électrique..., servent à alimenter l'appareil avec du courant électrique, en particulier la carte électronique des moyens de pilotage, typiquement un courant de tension comprise entre 110 et 220 V.

Tout ou partie de ces composants sont insérés dans une coque rigide formant la carcasse externe de l'appareil.

Ce type de ventilateur ne permet pas d'assurer un acheminement de l'oxygène issu de la première électrovanne 1 jusqu'au patient 11 lorsque la turbine 5 est arrêtée, que ce soit pour des questions d'économie d'énergie ou parce qu'elle est défaillante du fait d'un problème technique. En effet, on comprend aisément que le débit d'oxygène délivré par l'électrovanne 1 ne peut pas atteindre le patient 11 car, du fait de l'arrêt de la turbine 5, il remonte la canalisation d'amenée d'air 4 à contre-sens et s'échappe ensuite à l'atmosphère, étant donné que l'extrémité amont de la canalisation d'amenée d'air 4 est ouverte vers l'atmosphère.

Afin de résoudre ce problème, selon la présente invention, il est proposé de modifier l'architecture du ventilateur de la Figure 1 afin de pouvoir continuer à délivrer du gaz au patient 11 et donc d'assurer une ventilation dudit patient 11, même lorsque la turbine 5 est arrêtée.

La solution selon la présente invention est illustrée en Figure 2.

Comme on le voit, l'architecture globale et le principe de fonctionnement du ventilateur selon la présente invention sont identiques à ceux de la Figure 1. On se reportera donc aux explications données ci-dessus.

Toutefois, selon la présente invention, afin de résoudre le problème susmentionné et permettre d'assurer un acheminement de l'oxygène issu de la première électrovanne 1 jusqu'au patient 11 même lorsque la turbine 5 est arrêtée, il a été ajouté sur le conduit d'amenée d'air 4 du ventilateur, un organe d'obturation de conduit 14 piloté, c'est-à-dire commandé, par la carte de commande 9 de manière à obturer le conduit d'amenée d'air 4 et empêcher toute circulation de gaz en direction de l'orifice d'entrée d'air 4a du conduit 4, lequel communique fluidiquement avec l'atmosphère ambiant.

L'organe d'obturation de conduit 14 est avantageusement un dispositif du type actionneur 14 à deux positions de fonctionnement, à savoir une position ouverte et une position fermée. Bien entendu, tout autre dispositif fonctionnant sur le principe ouvert/fermé peut être utilisé.

En fonctionnement normal, c'est-à-dire lorsque la turbine 5 fonctionne, l'actionneur 14 est normalement en position ouverte. Il présente une résistance à l'écoulement d'air négligeable, par exemple de l'ordre de 2 cm d'eau pour un débit gazeux de 200 l/min. Le fonctionnement du ventilateur est alors identique à celui d'un ventilateur de l'état de l'art, tel que décrit en Figure 1.

Par contre, en cas d'arrêt de la turbine 5, la carte de commande 9 pilote l'actionneur 14 pour le faire passer en position fermée de sorte que le gaz (i.e., $O_2$) délivré par l'électrovanne 1 ne puisse plus remonter dans la canalisation 4 mais puisse, à l'inverse, s'écouler à travers la turbine 5 pour atteindre ensuite le patient 11, via les conduits 8 et 10a.

De façon simultanée, la carte de commande 9 pilote également l'électrovanne 1 de manière à alterner :

- d'une part, des phases de délivrance de gaz correspondant à des phases inspiratoire du patient 11, et
- d'autre part, des phases de non-délivrance de gaz correspondant à des phases expiratoire du patient 11.

Pour ce faire, la carte de commande 9 pilote l'électrovanne 1 en réponse à des signaux de débit ou de pression provenant des capteurs de débit 6 ou de pression 7, dans le cadre d'une ventilation volumétrique ou barométrique, respectivement.

Pendant les phases de délivrance de gaz, de l'oxygène est autorisé par l'électrovanne 1 à circuler vers le ventilateur 5, à un débit de délivrance D donné.

Par contre, pendant les phases de non-délivrance de gaz, l'électrovanne 1 empêche toute circulation d'oxygène vers le ventilateur 5 ou limite cette circulation à un débit faible d, tel que d < D. Pendant la phase expiratoire, on peut avoir par exemple un débit limité ou réduit d compris entre 1 et 10 l/min, i.e. le débit de biais.

Un capteur de pression additionnel 15 est par ailleurs agencé sur le conduit d'amenée d'air 4 pour y mesurer la pression et ainsi permettre de s'assurer de l'étanchéité du système, notamment de l'actionneur 14 faisant office d'organe d'obturation de conduit.

Il apparaît par ailleurs qu'une telle architecture peut être avantageusement utilisée afin de simplifier et fiabiliser la capacité du ventilateur 5 à effectuer un autodiagnostic.

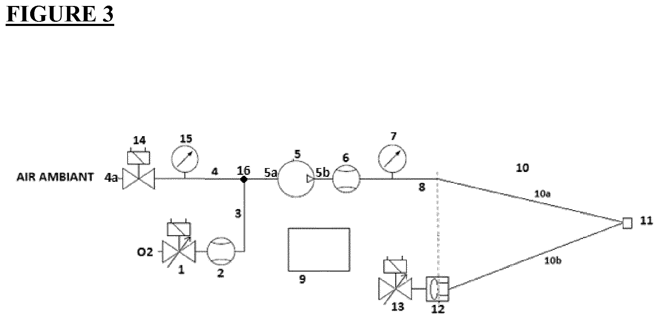

Pour ce faire, on obture d'abord la pièce Y du circuit patient 10 avec un bouchon (situé à la place du patient en 11) de manière à permettre à l'ensemble du système formé par le ventilateur 5 et le circuit patient 10 d'être étanche, comme illustré en Figure 3.

Ensuite, une première phase consiste notamment à vérifier que le système est étanche et notamment que l'actionneur 14 rempli bien sa fonction lorsque piloté en position fermée par la carte de commande 9.

A cette fin, la carte de commande 9 pilote l'électrovanne 13 de manière à fermer la membrane 12 et rendre cette portion du circuit étanche. Dans le même temps, la carte de commande 9 pilote la turbine 5 afin d'élever la pression dans le ventilateur et le circuit patient 10 à un niveau approprié, par exemple 50 cm d'eau au-dessus de la pression ambiante (l'information de pression est issue du capteur 7 et transformée par la carte de commande 9).

Lorsque cette pression est atteinte la carte de commande 9 pilote l'actionneur 14, initialement en position ouverte, vers une position fermée. L'électrovanne 1 est elle-même pilotée par la carte de commande 9 en position fermée.

Tous les ports d'entrée et sortie du ventilateur, représentées par l'actionneur 14, l'électrovanne 1, le bouchon et la membrane 12, sont maintenus en position fermée et le système est alors clos. La carte de commande 9 arrête alors la turbine 5 tout en réalisant dans le même temps la lecture du signal de pression issu du capteur 15.

Cette lecture est réalisée pendant 10 secondes environ après arrêt de la turbine 5 : à l'issue de ces 10 secondes, si la valeur de pression reste au-dessus de 90% de la valeur initialement lue, le système dans son ensemble est considéré comme étanche, c'est-à-dire que ses différents ports d'entrée et sortie sont fonctionnels, notamment l'actionneur 14.

Avoir l'assurance d'une telle intégrité permet notamment de réaliser un contrôle de cohérence des capteurs de débit 2 et 6. Pour ce faire, l'actionneur 14 est maintenu par la carte de commande 9 en position fermée. Cette même carte de commande 9 pilote l'électrovanne 13 de telle manière à ouvrir la membrane 12 et permettre la circulation d'un débit à travers les branches inspiratoire 10a et expiratoire 10b du circuit patient 10. La carte de commande pilote alors l'électrovanne 1 afin de générer un ou plusieurs débits étalons en se basant sur la valeur issue du capteur de débit 2 et de comparer cette ou ces valeurs à la valeur de débit récupérée du capteur 6 par la carte de commande 9. Si la ou les valeurs de débit sont cohérentes entre elles, il apparaît que les deux capteurs 2 et 6 sont fonctionnels, ce qui garantit la qualité de ventilation du patient 11.

L'appareil de ventilation de l'invention est utilisable pour traiter toute pathologie respiratoire nécessitant une administration d'air ou d'un mélange d'air enrichi en oxygène à un patient atteint de ladite pathologie respiratoire.

**Revendications**

1. Appareil d'assistance respiratoire comprenant :

   - une turbine (5) à moteur électrique comprenant une entrée de gaz (5a) et une sortie de gaz (5b), la sortie de gaz (5b) de la turbine (5) étant en communication fluidique avec un circuit patient (10), la sortie de gaz (5b) de la turbine (5) étant reliée fluidiquement au circuit patient (10) par l'intermédiaire d'une ligne d'alimentation en gaz (8),
   - un conduit d'amenée d'air (4) comprenant un orifice d'entrée d'air (4a) communiquant fluidiquement avec l'atmosphère ambiant, ledit conduit d'amenée d'air (4) par ailleurs relié fluidiquement à l'entrée de gaz (5a) de la turbine (5),
   - un conduit d'alimentation en oxygène (3) venant se raccorder au conduit d'amenée d'air (4), en un site de raccordement (16) situé en amont de l'entrée de gaz (5a) de la turbine (5), le conduit d'alimentation en oxygène (3) étant en communication fluidique avec le conduit d'amenée d'air (4),
   - des moyens de pilotage (9) coopérant avec la turbine (5) de manière à pouvoir détecter tout arrêt de fonctionnement de ladite turbine (5), et
   - un organe d'obturation de conduit (14) agencé sur le conduit d'amenée d'air (4) entre l'orifice d'entrée d'air (4a) et le site de raccordement (16) du conduit d'alimentation en oxygène (3) au conduit d'amenée d'air (4),

   **caractérisé en ce que** :

   - les moyens de pilotage (9) sont configurés pour détecter un arrêt du fonctionnement de la turbine (5) et pour piloter l'organe d'obturation de conduit (14) de manière à obturer le conduit d'amenée d'air (4) et à empêcher toute circulation de gaz vers l'orifice d'entrée d'air (4a) lorsque lesdits moyens de pilotage (9) détectent un arrêt du fonctionnement de la turbine (5),
   - une première électrovanne (1) est agencée sur le conduit d'alimentation en oxygène (3),
   - la ligne d'alimentation en gaz (8) comprend un capteur de débit (6) et un capteur de pression (7), et
   - les moyens de pilotage (9) sont en outre configurés pour commander la première électrovanne (1), en cas d'arrêt de la turbine (5), en réponse à des signaux provenant du capteur de débit (6) ou du capteur de pression (7) de manière à ajuster le débit d'oxygène circulant dans ledit conduit d'alimentation en oxygène (3) en alternant :

     . des phases de délivrance de gaz durant lesquelles de l'oxygène peut traverser la première électrovanne (1) à un débit de délivrance (D) donné, et
     . des phases de non-délivrance de gaz durant lesquelles de l'oxygène ne traverse pas la première électrovanne (1) ou la traverse à un débit limité (d).

2. Appareil selon la revendication précédente, **caractérisé en ce que** l'organe d'obturation de conduit (14) comprend un actionneur à deux positions de fonctionnement comprenant :

   - une position ouverte dans laquelle le gaz peut circuler dans le conduit d'amenée d'air (4), et
   - une position fermée dans lequel le gaz ne peut pas circuler dans le conduit d'amenée d'air (4) et sortir dudit conduit d'amenée d'air (4) par l'orifice d'entrée d'air (4a).

3. Appareil selon la revendication 1, **caractérisé en ce que** les moyens de pilotage (9) sont configurés pour piloter la première électrovanne (1) lors des phases de non-délivrance de gaz, de manière à ce que de l'oxygène traverse la première électrovanne (1) à un débit (d) limité inférieur ou égal à 10 l/min.

4. Appareil selon la revendication 1, **caractérisé en ce que** les moyens de pilotage (9) sont configurés pour piloter la première électrovanne (1), lors des phases de délivrance de gaz, de manière à ce que de l'oxygène traverse la première électrovanne (1) à un débit de délivrance (D) inférieur ou égal à 200 l/min.

5. Appareil selon l'une des revendications précédentes, **caractérisé en ce que** le conduit d'alimentation en oxygène (3) comprend un capteur de débit (2) additionnel.

6. Appareil selon la revendication 1, **caractérisé en ce que** le conduit d'amenée d'air (4) comprend un capteur de pression (15) additionnel.

7. Appareil selon l'une des revendications précédentes, **caractérisé en ce que** les moyens de pilotage (9) comprennent au moins une carte électronique de commande.

8. Appareil selon les revendications 5, 6 et 7, **caractérisé en ce que** la carte électronique de commande est configurée pour traiter des signaux provenant des capteurs de débit (2, 6) et de pression (7, 15).

9. Appareil selon la revendication 5, **caractérisé en ce que** la première électrovanne (1) est agencée sur le conduit d'alimentation en oxygène (3), entre l'entrée du conduit d'alimentation (3) en oxygène à laquelle vient se raccorder fluidiquement une source d'oxygène, et le capteur de débit (2) du conduit d'alimentation en oxygène (3).

10. Installation de fourniture de gaz à un patient comprenant un appareil d'assistance respiratoire selon l'une des revendications précédentes, **caractérisée en ce que** le circuit patient (10) est relié fluidiquement à une interface respiratoire.

11. Installation de fourniture de gaz selon la revendication 10, **caractérisée en ce que** l'interface respiratoire est un masque, une sonde trachéale ou des lunettes respiratoires.

12. Installation de fourniture de gaz selon la revendication 10 ou 11, **caractérisée en ce que** le circuit patient (10) comprend une branche inspiratoire (10a) et une branche expiratoire (10b) comprenant une vanne expiratoire à membrane expiratoire (12) commandée par une seconde électrovanne (13), ladite seconde électrovanne (13) étant pilotée par les moyens de pilotage (9).

13. Installation de fourniture de gaz selon la revendication 12, **caractérisée en ce que** la seconde électrovanne (13) est de type proportionnel.


**Patentansprüche**

1. Einrichtung zur Atmungsunterstützung, umfassend:

- eine Turbine (5) mit Elektromotor, die einen Gaseinlass (5a) und einen Gasauslass (5b) umfasst, wobei sich der Gasauslass (5b) der Turbine (5) in fluider Kommunikation mit einem Patientenkreislauf (10) befindet, wobei der Gasauslass (5b) der Turbine (5) mittels einer Gasversorgungsleitung (8) mit dem Patientenkreislauf (10) fluidisch verbunden ist,
- einen Luftzufuhrkanal (4), der eine Lufteinlassöffnung (4a) umfasst, die mit der Raumatmosphäre fluidisch kommuniziert, wobei der Luftzufuhrkanal (4) darüber hinaus mit dem Gaseinlass (5a) der Turbine (5) fluidisch verbunden ist,
- einen Sauerstoffversorgungskanal (3), der an den Luftzufuhrkanal (4) an einer Anschlussstelle (16) angeschlossen ist, die sich stromaufwärts von dem Gaseinlass (5a) der Turbine (5) befindet, wobei sich der Sauerstoffversorgungskanal (3) mit dem Luftzufuhrkanal (4) in fluider Kommunikation befindet,
- Steuermittel (9), die mit der Turbine (5) so zusammenwirken, dass jeglicher Betriebsstopp der Turbine (5) erfasst werden kann, und
- ein Kanalverschlussorgan (14), das auf dem Luftzufuhrkanal (4) zwischen der Lufteinlassöffnung (4a) und der Anschlussstelle (16) des Sauerstoffversorgungskanals (3) an den Luftzufuhrkanal (4) angeordnet ist,

**dadurch gekennzeichnet, dass**:

- die Steuermittel (9) konfiguriert sind, um einen Betriebsstopp der Turbine (5) zu erfassen und das Kanalverschlussorgan (14) so zu steuern, dass der Luftzufuhrkanal (4) verschlossen wird und jegliche Gaszirkulation in die Lufteinlassöffnung (4a) verhindert wird, wenn die Steuermittel (9) einen Betriebsstopp der Turbine (5) erfassen,
- ein erstes Magnetventil (1) auf dem Sauerstoffversorgungskanal (3) angeordnet ist,
- die Gasversorgungsleitung (8) einen Durchflusssensor (6) und einen Drucksensor (7) umfasst, und
- die Steuermittel (9) weiter konfiguriert sind, um das erste Magnetventil (1) im Fall eines Stopps der Turbine (5) als Antwort auf Signale, die von dem Durchflusssensor (6) oder dem Drucksensor (7) stammen, so zu regeln, dass der Sauerstoffdurchfluss, der in dem Sauerstoffversorgungskanal (3) zirkuliert, eingestellt wird durch Abwechseln von:

- Gas-Abgabephasen, während derer Sauerstoff durch das erste Magnetventil (1) mit einem gegebenen Abgabedurchfluss (D) fließen kann, und
- Gas-Nichtabgabephasen, während derer Sauerstoff nicht durch das erste Magnetventil (1) fließt oder mit einem begrenzten Durchfluss (d) durch dieses fließt.

2. Einrichtung nach dem vorstehenden Anspruch, **dadurch gekennzeichnet, dass** das Kanalverschlussorgan (14) einen Steller mit zwei Betriebspositionen umfasst, umfassend:

- eine offene Position, in der das Gas in dem Luftzufuhrkanal (4) zirkulieren kann, und
- eine geschlossene Position, in der das Gas in dem Luftzufuhrkanal (4) nicht zirkulieren und aus dem Luftzufuhrkanal (4) durch die Lufteinlassöffnung (4a) austreten kann.

3. Einrichtung nach Anspruch 1, **dadurch gekennzeichnet, dass** die Steuermittel (9) konfiguriert sind, um das erste Magnetventil (1) während der Gas-Nichtabgabephasen so zu steuern, dass Sauerstoff durch das erste Magnetventil (1) mit einem begrenzten Durchfluss (d) fließt, der kleiner oder gleich 10 l/min ist.

4. Einrichtung nach Anspruch 1, **dadurch gekennzeichnet, dass** die Steuermittel (9) konfiguriert sind, um das erste Magnetventil (1) während der Gas-Abgabephasen so zu steuern, dass Sauerstoff durch das erste Magnetventil (1) mit einem Abgabedurchfluss (D) fließt, der kleiner oder gleich 200 l/min ist.

5. Einrichtung nach einem der vorstehenden Ansprüche, **dadurch gekennzeichnet, dass** der Sauerstoffversorgungskanal (3) einen zusätzlichen Durchflusssensor (2) umfasst.

6. Einrichtung nach Anspruch 1, **dadurch gekennzeichnet, dass** der Luftzufuhrkanal (4) einen zusätzlichen Drucksensor (15) umfasst.

7. Einrichtung nach einem der vorstehenden Ansprüche, **dadurch gekennzeichnet, dass** die Steuermittel (9) mindestens eine elektronische Reglerplatte umfassen.

8. Einrichtung nach Anspruch 5, 6 und 7, **dadurch gekennzeichnet, dass** die elektronische Reglerplatte konfiguriert ist, um Signale zu verarbeiten, die von den Durchfluss- (2, 6) und Drucksensoren (7, 15) stammen.

9. Einrichtung nach Anspruch 5, **dadurch gekennzeichnet, dass** das erste Magnetventil (1) auf dem Sauerstoffversorgungskanal (3) zwischen dem Einlass des Sauerstoffversorgungskanals (3), an den fluidisch eine Sauerstoffquelle angeschlossen wird, und dem Durchflusssensor (2) des Sauerstoffversorgungskanals (3) angeordnet ist.

10. Gasbereitstellungsanlage für einen Patienten, umfassend eine Einrichtung zur Atmungsunterstützung nach einem der vorstehenden Ansprüche,
**dadurch gekennzeichnet, dass** der Patientenkreislauf (10) mit einer Atmungsschnittstelle fluidisch verbunden ist.

11. Gasbereitstellungsanlage nach Anspruch 10, **dadurch gekennzeichnet, dass** die Atmungsschnittstelle eine Maske, ein Trachealtubus oder eine Atembrille ist.

12. Gasbereitstellungsanlage nach Anspruch 10 oder 11,
**dadurch gekennzeichnet, dass** der Patientenkreislauf (10) einen Einatmungszweig (10a) und einen Ausatmungszweig (10b) umfasst, der ein Ausatmungsventil mit Ausatmungsmembran (12) umfasst, das von einem zweiten Magnetventil (13) geregelt wird, wobei das zweite Magnetventil (13) von den Steuermitteln (9) gesteuert wird.

13. Gasbereitstellungsanlage nach Anspruch 12, **dadurch gekennzeichnet, dass** das zweite Magnetventil (13) proportionaler Art ist.

**Claims**

1. Breathing assistance device comprising:

- an electric motor turbine (5) comprising a gas inlet (5a) and a gas outlet (5b), the gas outlet (5b) of the turbine (5) fluidly communicating with a patient circuit (10), the gas outlet (5b) of the turbine (5) being fluidly connected to the patient circuit (10) by way of a gas supply line (8),

- an air inlet duct (4) comprising an air inlet orifice (4a) fluidly communicating with the ambient atmosphere, said air inlet duct (4) moreover fluidly connected to the gas inlet (5a) of the turbine (5),

- an oxygen supply duct (3) connected to the air inlet duct (4), in a connection site (16) situated upstream of the gas inlet (5a) of the turbine (5), the oxygen supply duct (3) fluidly communicating with the air inlet duct (4),

- controlling means (9) cooperating with the turbine (5) so as to be able to detect any stop in functioning of said turbine (5), and

- a duct blocking member (14) arranged on the air inlet duct (4) between the air inlet orifice (4a) and the connection site (16) of the duct for supplying oxygen (3) to the air inlet duct (4),

**characterised in that**:

- the controlling means (9) are configured to detect a stop in functioning of the turbine (5) and to control the duct blocking member (14) so as to block the air inlet (4) and to prevent any gas circulation towards the air inlet orifice (4a) when said controlling means (9) detect a stop in functioning of the turbine (5),

- a first solenoid valve (1) is arranged on the oxygen supply duct (3),

- the gas supply line (8) comprises a flow sensor (6) and a pressure sensor (7), and

- the controlling means (9) are furthermore configured to control the first solenoid valve (1), in case of the turbine (5) stopping, in response to signals coming from the flow sensor (6) or from the pressure sensor (7) so as to adjust the oxygen flow circulating in said oxygen supply duct (3) by alternating:

    - gas delivery phases during which the oxygen can pass through the first solenoid valve (1) at a given delivery flow (D), and

    - gas non-delivery phases during which the oxygen does not pass through the first solenoid valve (1) or passes through it at a limited flow (d).

2. Device according to the preceding claim, **characterised in that** the duct blocking member (14) comprises an actuator with two functioning positions comprising:

    - an open position wherein the gas can circulate in the air inlet duct (4), and

    - a closed position wherein the gas cannot circulate in the air inlet duct (4) and exit from said air inlet duct (4) through the air inlet orifice (4a).

3. Device according to claim 1, **characterised in that** the controlling means (9) are configured to control the first solenoid valve (1) during gas non-delivery phases, such that the oxygen passes through the first solenoid valve (1) at a limited flow (d) less than or equal to 10l/min.

4. Device according to claim 1, **characterised in that** the controlling means (9) are configured to control the first solenoid valve (1), during the gas delivery phases, such that the oxygen passes through the first solenoid valve (1) at a delivery flow (D) less than or equal to 200l/min.

5. Device according to one of the preceding claims, **characterised in that** the oxygen supply duct (3) comprises an additional flow sensor (2).

6. Device according to claim 1, **characterised in that** the air inlet duct (4) comprises an additional pressure sensor (15).

7. Device according to one of the preceding claims, **characterised in that** the controlling means (9) comprise at least one electronic control board.

8. Device according to claims 5, 6 and 7, **characterised in that** the electronic control board is configured to process signals coming from flow (2, 6) and pressure (7, 15) sensors.

9. Device according to claim 5, **characterised in that** the first solenoid valve (1) is arranged on the oxygen supply duct (3), between the inlet of the oxygen supply duct (3) to which is fluidly connected an oxygen source, and the flow sensor (2) of the oxygen supply duct (3).

**10.** Installation for supplying gas to a patient comprising a breathing assistance device according to one of the preceding claims, **characterised in that** the patient circuit (10) is fluidly connected to a breathing interface.

**11.** Gas supply installation according to claim 10, **characterised in that** the breathing interface is a mask, an endotracheal tube or breathing goggles.

**12.** Gas supply installation according to claim 10 or 11, **characterised in that** the patient circuit (10) comprises an inspiratory limb (10a) and an expiratory limb (10b) comprising an expiratory valve with an expiratory membrane (12) controlled by a second solenoid valve (13), said second solenoid valve (13) being controlled by the controlling means (9).

**13.** Gas supply installation according to claim 12, **characterised in that** the second solenoid valve (13) is of the proportional type.

# FIGURE 1

# FIGURE 2

# FIGURE 3

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2013008444 A
- FR 2830454 A
- WO 0045883 A
- WO 2012032434 A
- US 20100787017 A
- US 5694926 A